# EUROPEAN PATENT SPECIFICATION

(11) **EP 3 833 743 B1**
(45) Date of publication and mention of the grant of the patent: **24.01.2024**
(21) Application number: 19847254.0
(22) Date of filing: 06.08.2019
(51) Int. Cl.: C12N 9/24, C12N 15/87, A61K 35/17, C07K 14/705, A61P 31/06

(54) **RECOMBINANT CD1-RESTRICTED T CELLS FOR TREATING TUBERCULOSIS**
REKOMBINANTE CD1-BESCHRÄNKTE T-ZELLEN ZUR BEHANDLUNG VON TUBERKULOSE
CELLULES T RECOMBINANTES RESTREINTES AU CD1 POUR LE TRAITEMENT DE LA TUBERCULOSE

(30) Priority: 08.08.2018 US 201862716292 P
(43) Date of publication of application: 16.06.2021
(73) Proprietor: NantBio, Inc., Culver City, California 90232 (US)
(72) Inventor: RICHARDSON, Wade, Culver City, California 90232 (US); WU, Ting, Culver City, California 90232 (US); SIELING, Peter, Culver City, California 90232 (US); NIAZI, Kayvan, Culver City, California 90232 (US)
(74) Representative: J A Kemp LLP
(86) International application number: PCT/US2019/045241
(87) International publication number: WO 2020/033366

(56) References cited:
- WO-A1-99/12562
- WO-A1-99/52547
- WO-A1-2016/070061
- KR-A- 20170 074 245
- US-A1- 2017 037 370
- US-A1- 2017 258 835
- Montamat-Sicotte Damien J ET AL: "A mycolic acid-specific CD1-restricted T cell population contributes to acute and memory immune responses in human tuberculosis infection", The Journal of clinical investigation, 1 June 2011 (2011-06-01), pages 2493-2503, XP055904925, United States DOI: 10.1172/JCI46216 Retrieved from the Internet: URL:https://www.ncbi.nlm.nih.gov/pmc/artic les/PMC3104771/pdf/JCI46216.pdf [retrieved on 2022-03-24]
- CHANCELLOR, A. et al.: "CD 1b-restricted GEM T cell responses are modulated by Mycobacterium tuberculosis mycolic acid meromycolate chains", Proceedings of the National Academy of Sciences, vol. 114, no. 51, 2017, pages E10956-E10964, XP055615726, DOI: 10.1073/pnas.1708252114
- GRAS, S. et al.: "T cell receptor recognition of CDlb presenting a mycobacterial glycolipid", Nature communications, vol. 7, 2016, pages 1-12, XP055683208, DOI: 10.1038/ncomms13257

## Description

### Field of the Invention

The field of the invention is cell-based immunotherapy, especially as it relates to use of genetically modified T cells that express at least a portion of a CDlb restricted T cell receptor.

### Background of the Invention

The background description includes information that may be useful in understanding the present invention. It is not an admission that any of the information provided herein is prior art or relevant to the presently claimed invention, or that any publication specifically or implicitly referenced is prior art.

CD1b presents wide variety of *Mycobacterium tuberculosis-derived* lipids, including mycolic acid, glucose monomycolate, glycerol monomycolate, diacylated sulfoglycolipids, lipoarabinomannan and phosphatidylinositol mannoside to corresponding CD1b restricted T cells. Mycolic acid constitutes a major lipid constituent of the *Mycobacterium tuberculosis* cell envelope and is typically considered *Mycobacterium tuberculosis* virulence factor.

Notably, mycolic acid-specific CD1b-restricted T cells have been found in blood as well as disease sites of *Mycobacterium tuberculosis-infected* individuals, and some CD1-restricted T cell lines from healthy or mycobacteria-infected individuals exhibited cytotoxicity and produced IFN-gamma and TNF-alpha, which are important for establishing protective immunity against *Mycobacterium tuberculosis.* More recently, various CD1b restricted T cell receptors have been isolated and characterized with respect to their interaction with CD1b and the bound lipid components (see Nature Communications 2017, DOI: 10.1038/ncomms13257).

In an effort to create a therapeutic composition to treat *Mycobacterium tuberculosis* infection, CD1 presented antigens can be prepared as a vaccine as described in CA2202680 or CA 2323733. Similarly, transgenic mice were generated that expressed human group 1 CD1 molecules (hCD1Tg) and a CD1b-restricted, mycolic-acid specific TCR in an effort to establish that CD1-targeting vaccines could be developed (eLife 2015; DOI: 10.7554/eLife.08525.001). Despite these conceptually simple and attractive approaches, effective vaccines have remained elusive. In yet another known approach, CDlb restricted T cells from various donors can be expanded to relatively large numbers (see e.g., US 2003/0153073), but such expansion generally requires elaborate processes and is typically not suitable in clinical settings.

WO 2016/070061 describes compositions and methods for generating modified cells with nucleic acid encoding a T cell receptor (TCR), a nucleic acid encoding a bispecific antibody, affinity molecule chimeric receptor, bispecific affinity molecule, or a chimeric ligand engineered activation receptor (CLEAR).

Thus, there remains a need for improved compositions, methods and uses of modified T cells with desired specificity against bacterial antigens, and especially against antigens related to *Mycobacterium tuberculosis.*

### Summary of The Invention

The inventive subject matter is directed to various uses of a recombinant primary T cells, that are transfected with a nucleic acid that encodes alpha and beta chains of a CDlb restricted T cell receptor. Advantageously, such recombinant T cells are suitable as a cell-based therapeutic for treatment of tuberculosis.

The invention thus provides a composition for use in the treatment of *Mycobacterium tuberculosis*-infected individuals, the composition comprising a plurality of primary T cells, wherein the T cells are stimulated with anti-CD3 and anti-CD28 antibodies; wherein the stimulated T cells are transfected with one or more recombinant nucleic acid vectors comprising nucleic acids encoding the alpha chain and beta chain of a CDlb restricted T cell receptor (TCR); and wherein the alpha chain comprises SEQ ID NO: 2 and the beta chain comprises SEQ ID NO: 4.

In one aspect of the inventive subject matter, the inventors contemplate a genetically modified T cell that includes a recombinant RNA molecule that encodes at least one of an alpha chain and a beta chain of a CDlb restricted T cell receptor. Preferably, the recombinant RNA molecule encodes both of the alpha chain and the beta chain of the clone 18 CDlb restricted T cell receptors. Therefore, preferred CDlb restricted T cell receptors will include at least one of the sequences according to SEQ ID NO: 1-4. Furthermore, preferred recombinant RNA molecules are bi- or polycistronic mRNA molecules that include at least one IRES sequence. Suitable T cells especially include autologous T cells and/or primary T cells.

In further contemplated aspects, the recombinant RNA molecule is at least temporarily coupled to a polymer particle that is optionally degradable within the T cell. For example, contemplated polymers of the polymer particle include a poly(β-amino ester) or a chitosan. Where desired, the polymer particle may further be coupled to a second mRNA that encodes an enzyme (*e.g*., chitosanase) capable of degrading the polymer of the polymeric particle.

A pharmaceutical composition that comprises a liquid carrier suitable for injection and a plurality of genetically modified T cells as described above suspended in the carrier is described. For example, contemplated pharmaceutical compositions may have at least 10⁷ genetically modified T cells per transfusion unit, and/or the liquid carrier has a pH of at least 7.0. Consequently, in still another aspect of the inventive subject matter, the inventors also contemplate method of treating an individual diagnosed with tuberculosis. Such methods will typically include a step of administering to the individual a plurality of genetically modified T cells as described above in an amount effective to treat tuberculosis (*e.g*., at least 10⁷ genetically modified T cells).

Therefore, uses of a genetically modified T cell in the treatment of tuberculosis are also contemplated where the genetically modified T cell comprises a recombinant RNA molecule that encodes at least one of an alpha chain and a beta chain of a CDlb restricted T cell receptor. The CDlb restricted T cell receptor is a clone 18 CDlb restricted T cell receptor, and/or the genetically modified T cell is an autologous T cell.

A method of generating a genetically modified T cell is described. Such methods will typically include a step of obtaining or providing a plurality of T cells, and stimulating the plurality of T cells; and a further step of transfecting the stimulated T cells with a recombinant RNA molecule that encodes at least one of an alpha chain and a beta chain of a CDlb restricted T cell receptor.

Preferably, the plurality of T cells are primary T cells, which may or may not be autologous cells. It is further generally preferred that the plurality of T cells are stimulated prior to transfection (*e.g*., with CD3-CD28 modified beads). Moreover, it is contemplated that transfection of the stimulated T cells uses a plurality of polymeric particles that are coupled to the recombinant RNA molecule. It should be noted that the polymeric particles may be all of the same type, or comprise at least two different types of polymeric particles.

Additionally, it is contemplated that the plurality of polymeric particles are degradable in the stimulated T cells, and/or that the polymeric particles lack targeting moieties that specifically target the particles to a component of the T cell. Suitable polymer materials for contemplated particles include poly(β-amino esters) and a chitosan. Where desired, the polymer particle may be further coupled to (*e.g*., coated with) a second and distinct mRNA, which may encode an enzyme capable of degrading the polymer of the polymeric particle.

Preferably, transfection is performed such that at least 40%, or at least 50%, or at least 60% of all cells expressing the CDlb restricted T cell receptor are viable cells. Likewise, transfection is preferably performed such that at least 20% or at least 30% of all cells express the CDlb restricted T cell receptor. As noted earlier, the recombinant RNA molecule encodes the alpha chain and the beta chain of the CD1b restricted T cell receptor, and is preferably (but not necessarily) a clone 18 CD1b restricted T cell receptor. Therefore, the recombinant RNA may be configured as a bi- or polycistronic RNA and includes at least one IRES sequence. Among other suitable sequences, the CDlb restricted T cell receptor may comprise at least one of the sequences according to SEQ ID NO: 1-4.

Various objects, features, aspects and advantages of the inventive subject matter will become more apparent from the following detailed description of preferred embodiments, along with the accompanying drawing.

### Brief Description of the Drawing

Figure 1 is a graph depicting exemplary results for T cell transfections of stimulated and non-stimulated T cells using targeting and non-targeting PBAE nanoparticles.
Figure 2 is a graph depicting exemplary results for T cell transfections of stimulated T cells using targeting and non-targeting PBAE nanoparticles.
Figure 3 is a graph depicting exemplary results for T cell transfections of stimulated and non-stimulated primary T cells using targeting and non-targeting PBAE nanoparticles.
Figure 4 is a graph depicting exemplary results for T cell stimulation using CD3-CD28 beads.
Figure 5 is a graph depicting exemplary results for GFP expression in stimulated and non-stimulated primary T cells using targeting and non-targeting PBAE nanoparticles.
Figure 6 is a graph depicting exemplary results for viability and GFP expression of stimulated and non-stimulated primary T cells using targeting and non-targeting PBAE nanoparticles.
Figure 7 is an exemplary schematic of chitosan/chitosanase nanoparticle transfections.
Figure 8 is one graph depicting exemplary results for viability and GFP expression in HEK293T cells using chitosan nanoparticles and various media.
Figure 9 is another graph depicting exemplary results for viability and GFP expression in HEK293T cells using chitosan nanoparticles and various media.
Figure 10 is a further graph depicting exemplary results for viability and GFP expression in HEK293T cells using chitosan nanoparticles and various media.
Figure 11 is an exemplary vector map for generation of chitosanase mRNA.
Figure 12 is a further graph depicting exemplary results for viability and GFP expression in HEK293T cells using various nanoparticles and media conditions.
Figure 13 is yet another graph depicting exemplary results for viability and GFP expression in HEK293T cells using various nanoparticles and media conditions.
Figure 14 depicts exemplary nucleic acid and protein sequences for a TCR 18 alpha chain.
Figure 15 depicts exemplary nucleic acid and protein sequences for a TCR 18 beta chain.

### Detailed Description

The inventors have now discovered that expression of a functional CDlb restricted T cell receptor in T cells, and especially human primary T cells, can be used to generate a cell-based therapeutic for the treatment of tuberculosis. Notably, using various protocols as described in more detail below, T cells (particularly primary T cells or autologous T cells) can be transfected at relatively high rate and viability.

Most typically, but not necessarily, T cells for transfection are obtained from a patient that is acutely infected with *Mycobacterium tuberculosis.* T cells are in most cases isolated from whole blood following protocols using magnetic bead separation (*e.g*., coated with antibodies against CD2 and/or CD3), and such isolated T cells can be further cultivated to expand the cell population. Transfection is preferably performed using particle associated mRNA that encodes both the α and β chains of a CDlb restricted T cell receptor (TCR) with high specificity to mycolic acid. For example an especially suitable TCR is clone 18 (*e.g*., Nature Communications 2016, DOI: 10.1038/ncomms13257) T cell receptor. Upon transfection with the RNA, the T cells are further cultured (and optionally further stimulated) and then administered to the same patient.

Of course, it should be appreciated that the particular nature of the infection of the patient with *Mycobacterium tuberculosis* is not limiting to the inventive subject matter, and all forms of infection, including pulmonary tuberculosis (*e.g*., latent/dormant, acute, and chronic) as well as extrapulmonary tuberculosis (*e.g*., pleural infection, meningeal infection, genitourinary infection, and infection of the lymphatic system) are expressly contemplated herein. Moreover, it should be noted that the patient need not be a human, but indeed may be any mammal that is subject to infection with *Mycobacterium tuberculosis.*

Additionally, it is contemplated that the CD1b receptor need not necessarily be limited to binding of mycolic acid, but that all other ligands (and especially microbial lipids) for CD1b presentation are also deemed suitable. For example, suitable sequence variations and changes in specificity are described elsewhere (*e.g.,* Nature Communications 2017, DOI: 10.1038/ncomms 13257). Therefore, numerous other microorganisms can also be targeted with the recombinant T cells as described herein. Likewise, it should be appreciated that numerous CD1 restricted TCR other than CD1b can be expressed in the T cells as noted herein, and especially contemplated subtypes include CD1a, CD1c, CD1d, and CD1e. Therefore, it should be noted that all restricted TCRs can be expressed in the T cells in a manner as described herein to so add/expand the pool of `innate immunity' in a patient.

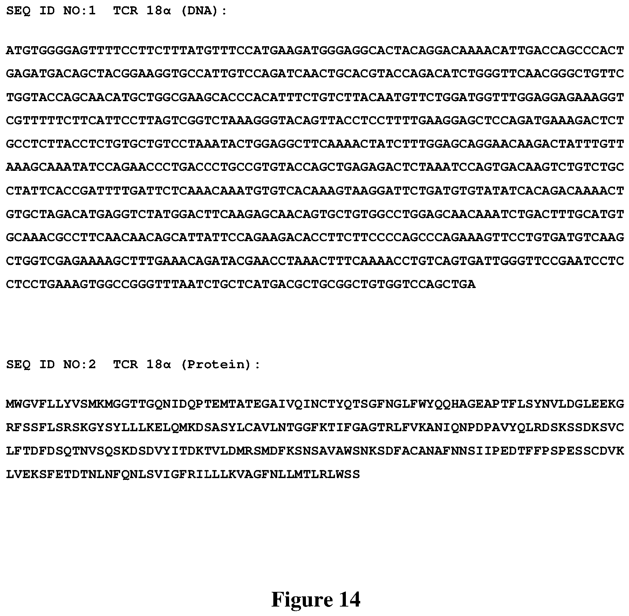

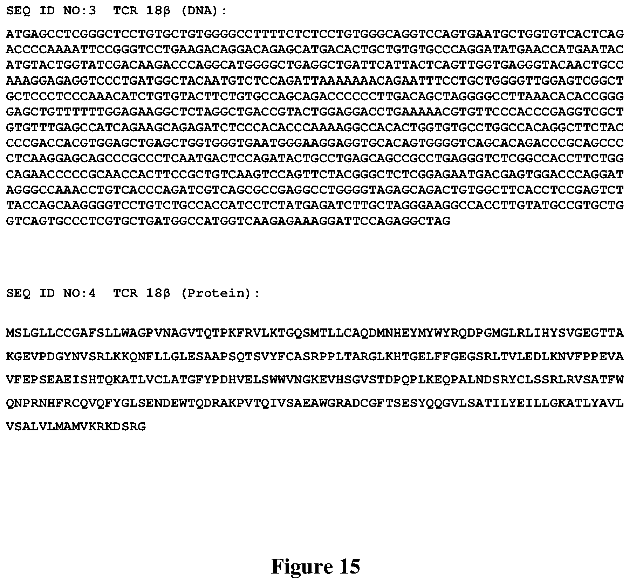

The recombinant TCR of the invention has or includes a sequence portion of the clone 18 CDlb restricted T cell receptor. Where the TCR will only include a portion of that sequence, any one or more of the Vα, Jα, Cα segments, and/or CDR1 and 2 in Vα and/or CDR3 for alpha chain are deemed suitable for use herein. Likewise, any one or more of the Vβ, Jβ, Dβ, Cβ segments, and/or CDR1 and 2 in Vβ and/or CDR3 for beta chain are deemed suitable for use herein. For example, particularly preferred sequences include those shown in Figures 14 and 15 (depicting SEQ ID Nos: 1-4), or any portion thereof. Therefore, recombinant nucleic acids encoding the CD1b restricted TCR include the alpha and/or beta chain of the clone 18 TCR, or have portions modified to adapt to specific lipid antigens (see *e.g.,* Nature Communications 2017, DOI: 10.1038/ncomms 13257; or Nat Immunol. 2013 July; 14(7): 706-713).

Furthermore, it should be noted that the nature of the T cells may also vary considerably, and suitable T cells for transfection include autologous (with respect to the patient) T cells, primary T cells, cultivated T cells, T cells from T cell culture (which will typically be irradiated before administration), CD4+ T cells, CD8+ T cells, and T cells with tissue compatibility to a recipient. Therefore, the manner of T cell isolation may vary considerably and suitable isolation protocols will include isolations via magnetic beads or FACS using antibodies against CD23, CD3, CD4, and/or CD25. Further known protocols are described elsewhere (see e.g., J Vis Exp. 2010; (40): 2017).

In less preferred aspects, it is also contemplated that the host cells are cells other than T cells, and especially preferred alternative cells include various cytotoxic immune competent cells such as NK cells or iNK cells. However, in such case, these cells will require further genetic modification (via transfection of other genetic engineering) to enable co-expression of one or more of the CD3 gamma, delta, epsilon, and zeta chain.

Regardless of the particular type of cell for transfection, it is typically preferred that the recombinant nucleic acid is an RNA, preferably constructed as a bicistronic or polycistronic RNA that includes one or more IRES sequences for coordinated expression of the alpha and beta chain. However, monocistronic RNA is also deemed suitable, albeit less preferred. Also less preferred transfections include those with DNA (whether as gene edited modification, or as extrachromosomal). Still further contemplated transfection nucleic acids include viral vectors.

Transfection of the T cells is performed on stimulated (*e.g*., using CD3/CD28 dynabeads) T cells using one or more types of nanoparticles that are coupled to one or more RNA molecules. The T cells are stimulated with anti-CD3 and anti-CD28 antibodies. In particularly preferred aspects, the nanoparticles are degradable within the T cells, typically in an enzymatic manner. For example, especially preferred polymers of the nanoparticles are poly(β-amino esters) (see e.g., Methods Mol Biol. 2009 ; 480: 53-63) and chitosan (see *e.g.,* Mol. Pharmaceutics 2017, 14, 2422-2436; or International Journal of Nanomedicine 2010:5 473-481). As will be readily appreciated, these nanoparticles can be targeted (*e.g*., with antibodies or fragments of antibodies binding CD3) or 'naked' (untargeted) as is discussed in more detail below. Alternatively, transfection of the T cells can also be performed using electroporation (see *e.g.,* Molecular Therapy Vol. 13, No. 1, p151-159, January 2006), or chemical transfection such as Nucleofactor or Amaxa kits (commercially available from Lonza, Allendale, NJ, USA).

Once transfected T cells are prepared, such cells are preferably formulated for transfusion to a patient, typically in an aqueous carrier suitable for injection. For example, a typical solution for transfusion will include at least 10⁶, or at least 10⁷, or at least 10⁸, or at least 10⁹ transfected cells. Thus, pharmaceutical compositions comprising transfected T cells and use of such cells in pharmaceutical formulations (especially for treatment of (myco)bacterial infection) are deemed appropriate.

### Examples

The following description provides the person of ordinary skill in the art exemplary guidance for preparation and use of the recombinant cells presented herein. However, the examples should not be construed as limiting the inventive subject matter.

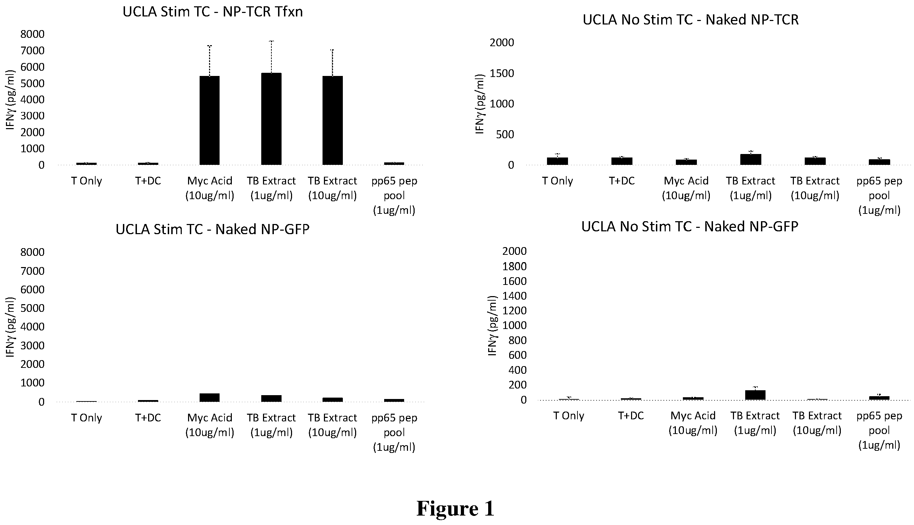

To investigate the influence of T cell stimulation and type of transfection on the T cell response to various antigens, T cells were separately transfected with 'naked' non-targeting PBAE (poly(β-amino ester)) nanoparticles that were associated with two types of RNA: RNA encoding a clone 18 TCR (bicistronic construct with clone 18 TCR alpha and beta chain) and RNA encoding GFP (green fluorescent protein). Stimulation, in this case, was achieved by culturing isolated T cells with a cocktail of anti-CD3 and anti-CD28 antibodies (Immunocult Human CD3/CD28 T Cell Activator, Stemcell Technologies) for 24 hours followed by another 24 hours in unsupplemented culture medium prior to transfection. PBAE-RNA nanoparticles were prepared by first dissolving both PBAE and RNA separately in an acidic sodium acetate buffer, then adding the PBAE solution to the RNA solution at the appropriate ratio and rapidly mixing them by vortex. After allowing 5 minutes for nanoparticle formation they were added to the T cells in culture for 24 hours. These transfected T cells were then, in some cases, co-cultured with human peripheral blood monocyte-derived dendritic cells with the addition, in some additional cases, of ligands for the clone 18 TCR. These were either mycolic acid or an extract from *Mycobacterium tuberculosis.* Following approximately 24 hours of culture the amount of interferon gamma present in the culture media was measured by enzyme-linked immunosorbent assay (ELISA). As can be readily seen from **Figure 1****,** stimulation of the T cells had significant effect in terms of interferon gamma secretion by the transfected T cells with all tested CDlb ligands (top panels; mycolic acid and TB extract). No effect was seen on control peptide pp65. No effect with respect to interferon gamma secretion was also observed in the control transfection with GFP. Here, T only denotes T cells only as negative control; T+DC denotes T cells and dendritic cells; Myc Acid denotes addition of mycolic acid as a single identified Cd1b ligand, whereas TB extract denotes a complex mixture of M. tuberculosis. The pp65 peptide was used as a non-binding control.

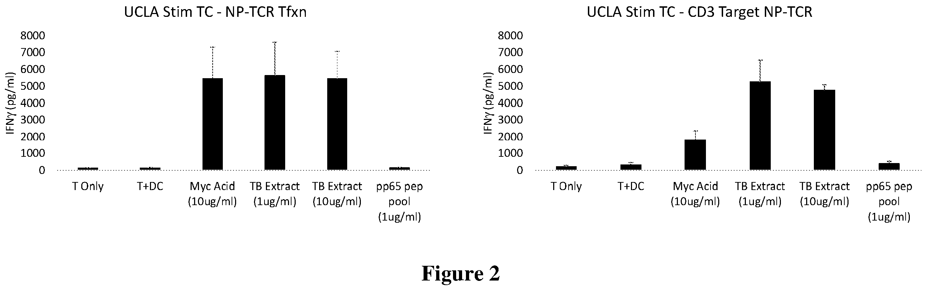

The inventors also investigated whether targeting of the nanoparticles to the T cells (here CD3 targeting) had any effect of the ligand dependent interferon gamma secretion. Notably, as can be taken from **Figure 2****,** transfection of stimulated T cells using CD3 targeted nanoparticles (PBAE) provided similar effects for TB extracts, but a somewhat reduced effect for mycolic acid. More specifically, in order to target the nanoparticles, anti-CD3 monoclonal antibodies were chemically conjugated to polyanionic polyglutamic acid (PGA), and these conjugates were electrostatically adsorbed to the cationic naked PBAE-RNA particles. Thus, targeting did not provide a substantial advantage over untargeted transfection.

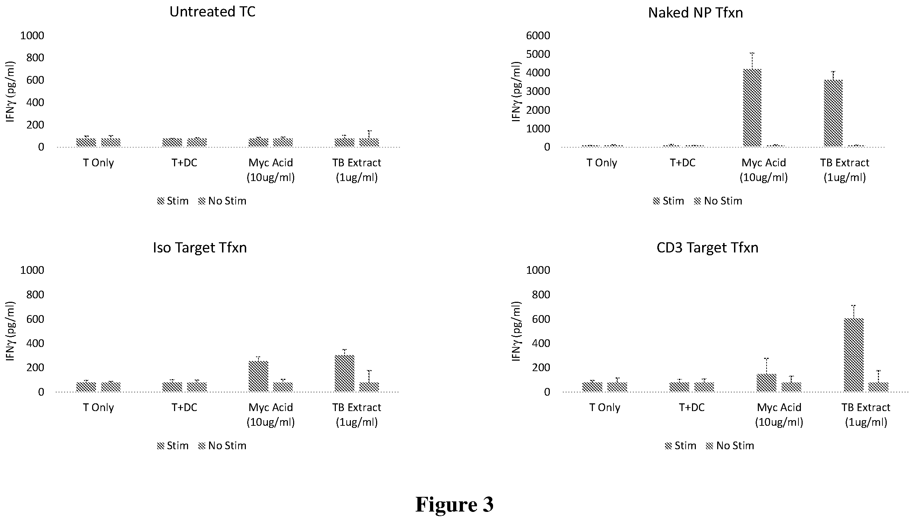

In yet further experiments, the inventors investigated effects of PBAE nanoparticle targeting where the T cells were stimulated or not stimulated. **Figure 3** depicts exemplary results in which stimulated T cells provided substantial interferon gamma secretion by the transfected T cells, with the same difference between targeted and non-targeted as observed in Figure 2. Control transfections had no substantial differences between stimulated and unstimulated (albeit some IFN-γ secretion in isotarget transfection). More specifically, isotarget particles were prepared by replacing the anti-CD3 antibody with an isotype antibody, which should not specifically target any cell surface markers..

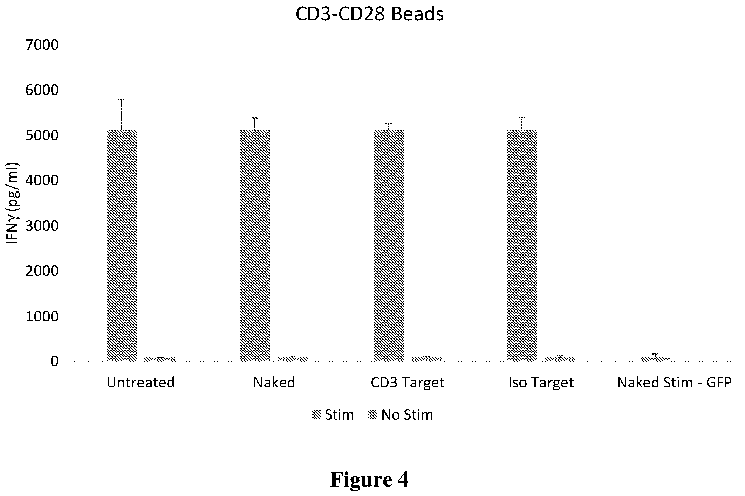

Stimulation of T cells was performed using CD3-CD28 dynabeads and resulted in similar responses for all types of transfections as can be seen in **Figure 4**. More specifically, Figure 4 shows the ELISA results from T cells that were treated with CD3-CD28 beads instead of CD1b/TB ligand presenting DCs, which was performed as a positive control to induce IPNγ production and to show that the T cells retained that functionality. Notably, only the stimulated cells responded even under these conditions.

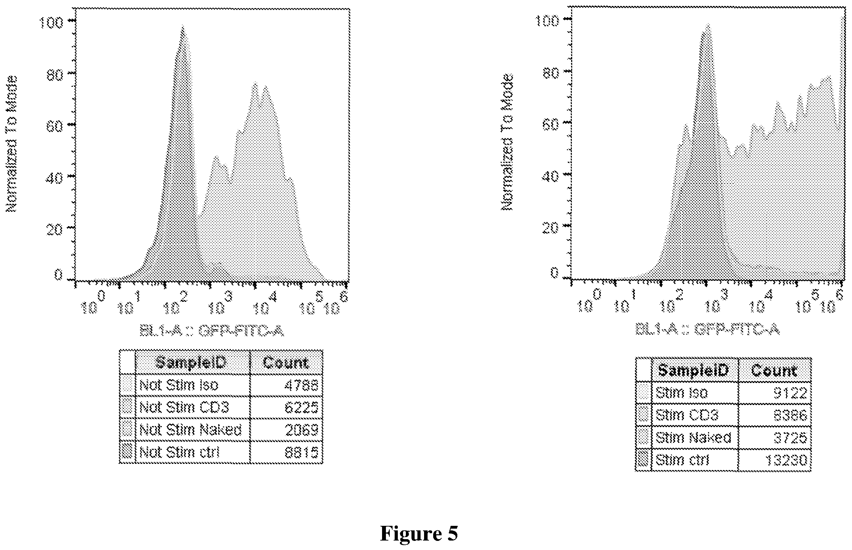

Functional expression of the recombinant RNA was also confirmed using RNA encoding GFP in a set of experiments in which transfection was done using stimulated and non-stimulated T cells. Representative results are seen in **Figure 5** where 'naked' PBAE nanoparticles provided the strongest fluorescence signal. More specifically, T cells were collected 24 hours after transfection as above and run on a Life Technologies Attune NxT flow cytometer to measure GFP expression.

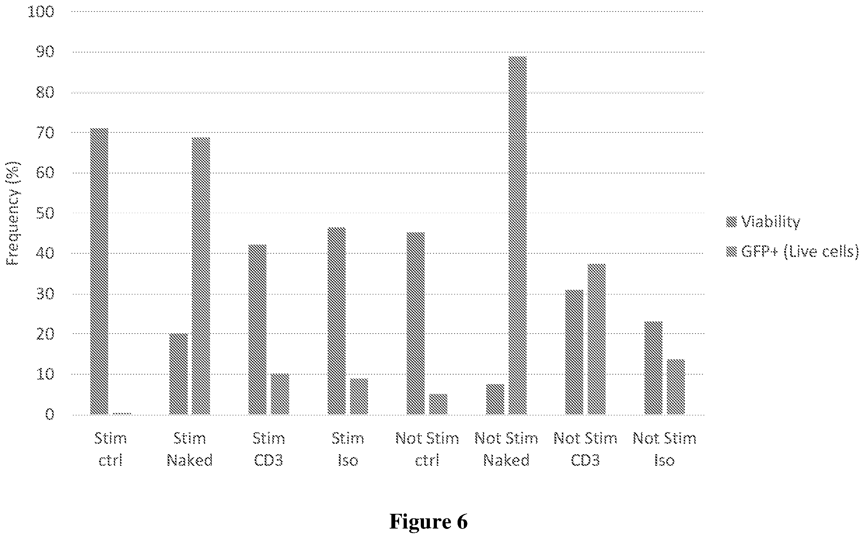

**Figure 6** depicts exemplary results from the same experiment as Figure 5. Results for viability and transfection of T cells are shown, once more comparing stimulated T cells with non-stimulated T cells using PBAE nanoparticles and RNA encoding GFP. In the graph of Figure 6, the first bar denotes the percentage of viable cells, while the second bar denotes the fraction of GFP expressing cells within the viable cell population.

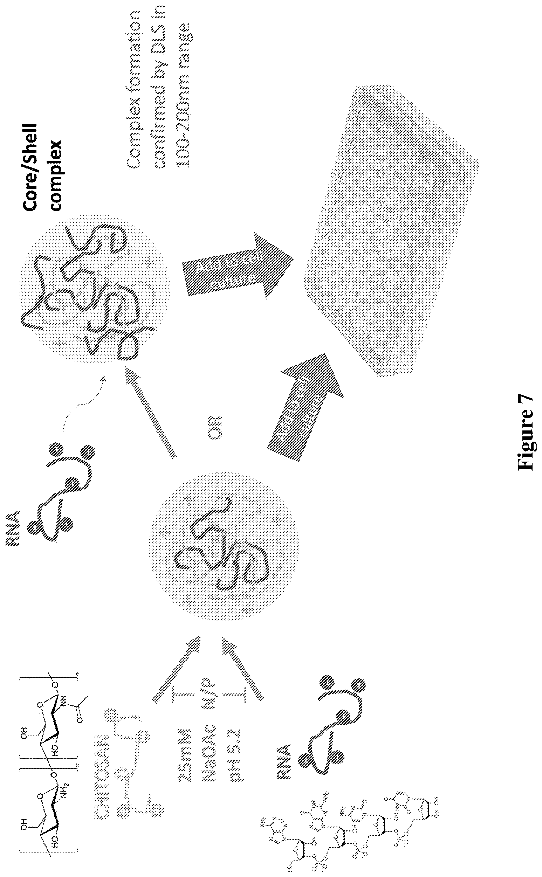

To determine the influence of the type of nanoparticles, the inventors also tested chitosan nanoparticles with associated RNA, where the chitosan particles were further optionally coated with RNA encoding chitosanase. An exemplary schematic workflow for preparation of such particles is depicted in **Figure 7**. Due to the positive charges of the chitosan, RNA readily associates with the chitosan during nanoparticle formation. Once formed (or during formation), the nanoparticle can be exposed to a second type of RNA (here RNA encoding chitosan) to so coat or otherwise associate the second RNA with the nanoparticle. It should be appreciated that such approach provides a nanoparticle that delivers the RNA to a cell where the RNA encodes the enzyme that degrades the nanoparticle. Thus, it should be appreciated that degradable nanoparticles can be prepared from a material that is selectively degradable by an enzyme. In such approach, the nanoparticle is then coated or otherwise associated with an RNA that encodes the material that is selectively degradable by the enzyme. Depending on the release kinetics of the RNA degradation kinetics of the nanoparticle can be controlled.

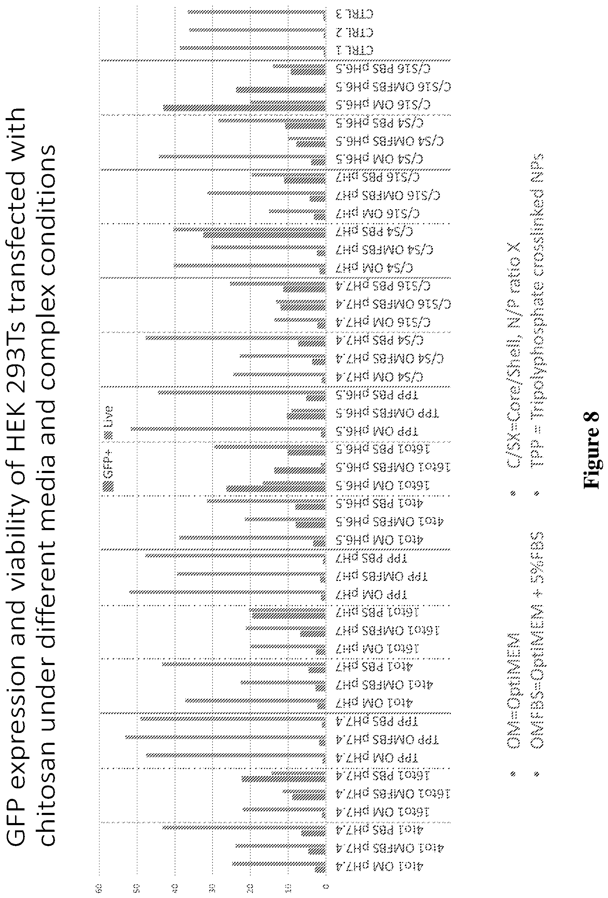

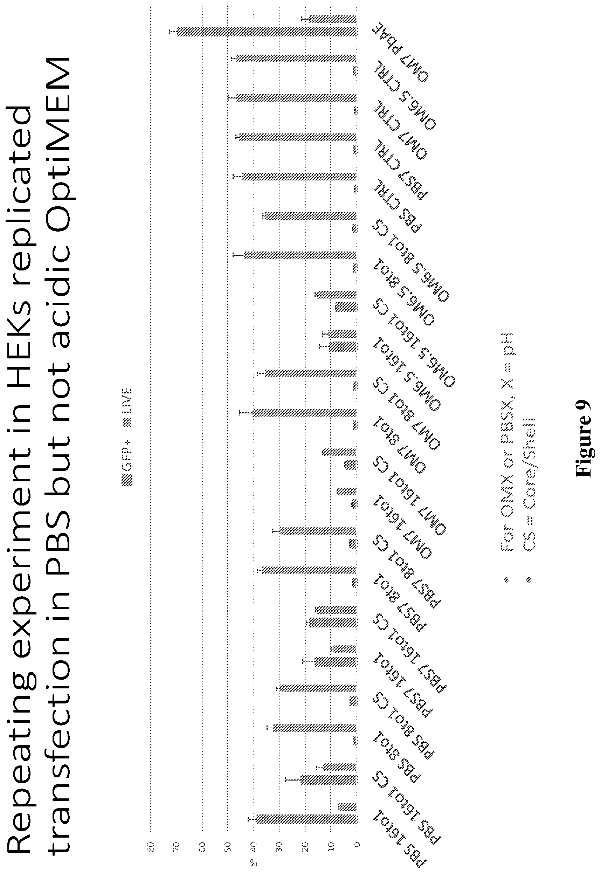

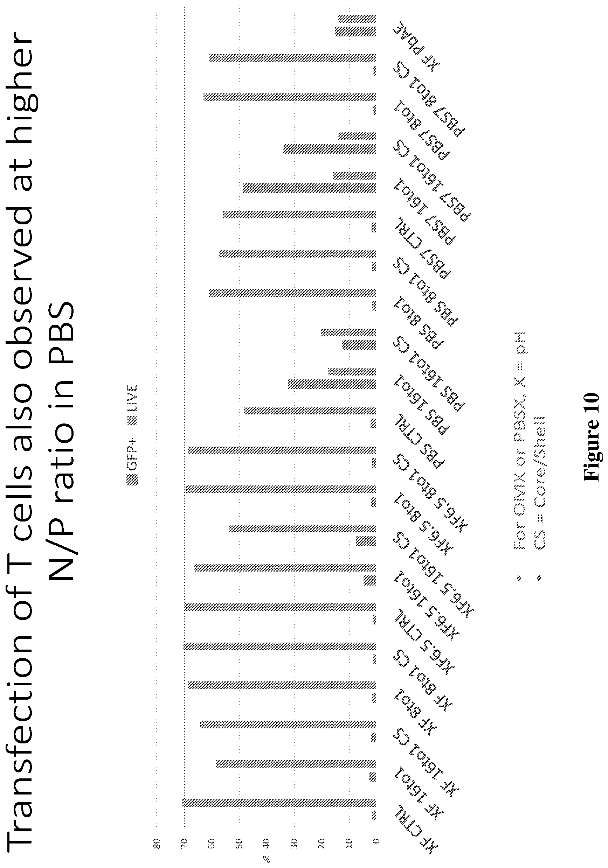

Exemplary results for nanoparticle RNA delivery using pure chitosan with single RNA (encoding GFP) and chitosan core/shell particles with GFP-encoding RNA comprising both the core and shell RNA component are shown in Figures 8-10 using HEK293T and T cells as indicated. More specifically, transfection efficiency and cell viability was compared by flow cytometry for transfections performed with a variety of nanoparticle and media conditions. Culture media used were phosphate buffered saline (PBS), OptiMEM (OM), and Stemcell Technologies' Immunocult -XF T Cell Expansion Medium (XF). The pH of the medium was also varied between 6 and 8. The N-to-P ratio of the chitosan-RNA complex was also varied 4 and 16 for both single complex and core/shell nanoparticles.

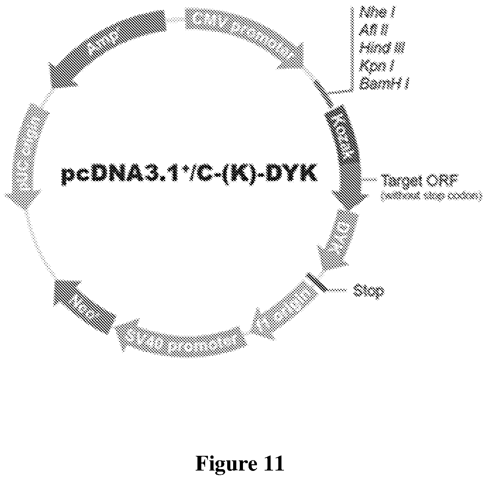

Chitosanase RNA was prepared by *in vitro* transcription reactions from a commercially available plasmid (pcDNA3.1+/c-(k)dyk) containing the chitosanase gene from *Colletotrichum higginsianum* IMI349063 (Gene Symbol: CH63R_01899) as is shown in **Figure 11**.

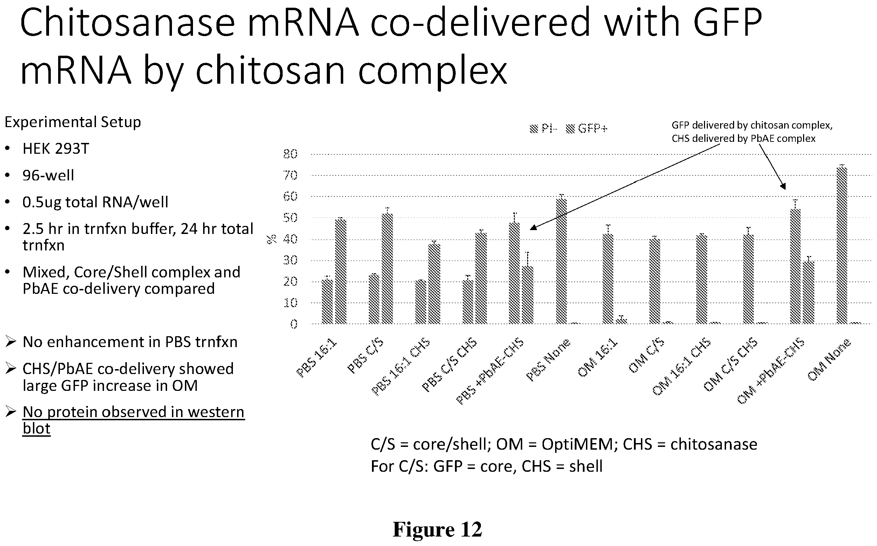

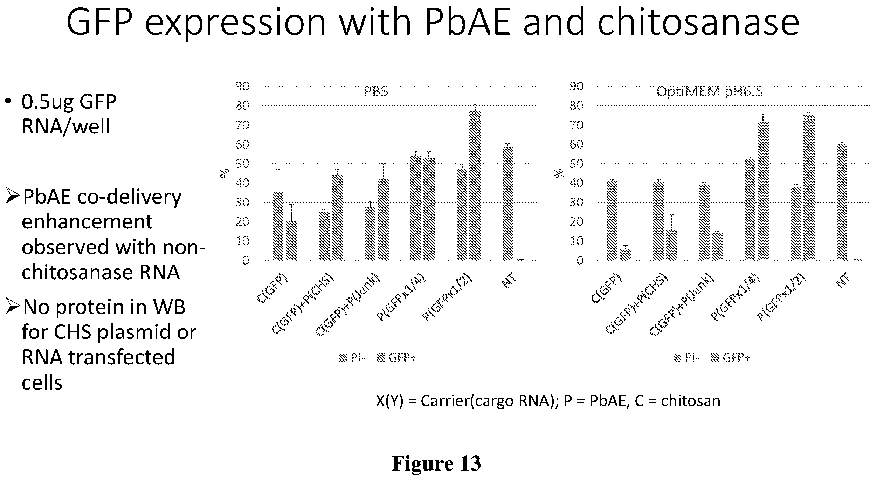

Exemplary results for co-delivery of chitosanase mRNA with GFP mRNA via core/shell chitosan complex and separate delivery are illustrated in **Figures 12-13**. The results in Figure 12 highlight the specific effects by media and nanoparticle composition, while the results in Figure 13 illustrate exemplary differences between the nanoparticle systems. More specifically, transfection efficiency and cell viability was compared by flow cytometry for HEK 293T cells transfected with GFP or chitosanase encoding RNA, with either chitosan or PBAE as the carrier, as well as chitosan core/shell configurations where GFP RNA was the core RNA component and chitosanase RNA was the shell RNA component.

It should be apparent to those skilled in the art that many more modifications besides those already described are possible without departing from the inventive concepts herein. The inventive subject matter, therefore, is not to be restricted except in the scope of the appended claims. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise. Where the specification claims refers to at least one of something selected from the group consisting of A, B, C .... and N, the text should be interpreted as requiring only one element from the group, not A plus N, or B plus N, etc.

## Claims

1. A composition for use in the treatment of *Mycobacterium tuberculosis-infected* individuals, the composition comprising a plurality of primary T cells, wherein the T cells are stimulated with anti-CD3 and anti-CD28 antibodies; wherein
the stimulated T cells are transfected with one or more recombinant nucleic acid vectors comprising nucleic acids encoding the alpha chain and beta chain of a CD1b restricted T cell receptor (TCR); and wherein the alpha chain comprises SEQ ID NO: 2 and the beta chain comprises SEQ ID NO: 4.

2. The composition for use according to claim 1, wherein:
(a) the plurality of T cells are autologous cells; and/or
(b) the plurality of T cells are stimulated with anti CD3-anti CD28 modified beads.

3. The composition for use according to claim 1 or 2, wherein the nucleic acid is recombinant RNA, and wherein transfection of the stimulated T cells uses a plurality of polymeric particles that are coupled to the recombinant RNA molecule.

4. The composition for use according to claim 3, wherein the plurality of polymeric particles comprise at least two different types of polymeric particles.

5. The composition for use according to claim 3 or claim 4, wherein:
(a) the plurality of polymeric particles are degradable in the stimulated T cells; or
(b) the plurality of polymeric particles lack targeting moieties that specifically target the particles to a component of the T cell.

6. The composition for use according to claim 3 or 4, wherein the polymer of the polymeric particle is a poly(β-amino ester) or a chitosan.

7. The composition for use according to claim 3 or 4, wherein the polymer particle is further coupled to a second and distinct mRNA.

8. The composition for use according to claim 7, wherein the second and distinct mRNA encodes an enzyme capable of degrading the polymer of the polymeric particle, optionally wherein the polymer of the polymer particle is a chitosan and wherein the enzyme is a chitosanase.

9. The composition for use according to any one of claims 1 to 8, wherein:
(a) the transfection is performed such that at least 20% of all cells express the CD1b restricted T cell receptor; or
(b) the transfection is performed such that at least 30% of all cells express the CD1b restricted T cell receptor; or
(c) the transfection is performed such that at least 40% of all cells expressing the CD1b restricted T cell receptor are viable cells; or
(d) the transfection is performed such that at least 50% of all cells expressing the CD1b restricted T cell receptor are viable cells; or
(e) the transfection is performed such that at least 60% of all cells expressing the CD1b restricted T cell receptor are viable cells.

10. The composition for use according to any one of the preceding claims, wherein at least 10⁷ genetically modified primary T cells are administered.

## Patentansprüche

1. Zusammensetzung zur Verwendung bei der Behandlung von mit *Mycobacterium tuberculosis* infizierten Individuen, wobei die Zusammensetzung eine Vielzahl von primären T-Zellen umfasst, wobei die T-Zellen mit Anti-CD3- und Anti-CD28-Antikörpern stimuliert sind; wobei
die stimulierten T-Zellen mit einem oder mehreren rekombinanten Nukleinsäurevektoren transfiziert sind, umfassend Nukleinsäuren, die die Alpha-Kette und Beta-Kette eines CD1b-beschränkten T-Zell-Rezeptors (TCR) kodieren; und wobei die Alpha-Kette SEQ ID NO: 2 umfasst und die Beta-Kette SEQ ID NO: 4 umfasst.

2. Zusammensetzung zur Verwendung nach Anspruch 1, wobei:
(a) die Vielzahl von T-Zellen autologe Zellen sind; und/oder
(b) die Vielzahl von T-Zellen mit Anti-CD3-AntiCD28-modifizierten Beads stimuliert sind.

3. Zusammensetzung zur Verwendung nach Anspruch 1 oder 2, wobei die Nukleinsäure rekombinante RNA ist und wobei Transfektion der stimulierten T-Zellen eine Vielzahl von Polymerteilchen verwendet, die an das rekombinante RNA-Molekül gekoppelt sind.

4. Zusammensetzung zur Verwendung nach Anspruch 3, wobei die Vielzahl von Polymerteilchen mindestens zwei unterschiedliche Arten von Polymerteilchen umfassen.

5. Zusammensetzung zur Verwendung nach Anspruch 3 oder Anspruch 4, wobei:
(a) die Vielzahl von Polymerteilchen abbaubar in den stimulierten T-Zellen sind; oder
(b) der Vielzahl von Polymerteilchen Zieleinheiten fehlen, die die Teilchen für eine Komponente der T-Zelle spezifisch anzielen.

6. Zusammensetzung zur Verwendung nach Anspruch 3 oder 4, wobei das Polymer des Polymerteilchens ein Poly(β-Aminoester) oder ein Chitosan ist.

7. Zusammensetzung zur Verwendung nach Anspruch 3 oder 4, wobei das Polymerteilchen ferner an eine zweite und eindeutige mRNA gekoppelt ist.

8. Zusammensetzung zur Verwendung nach Anspruch 7, wobei die zweite und eindeutige mRNA ein Enzym kodiert, das das Polymer des Polymerteilchens abbauen kann, wobei optional das Polymer des Polymerteilchens ein Chitosan ist und wobei das Enzym eine Chitosanase ist.

9. Zusammensetzung zur Verwendung nach einem der Ansprüche 1 bis 8, wobei:
(a) die Transfektion durchgeführt wird, sodass mindestens 20 % aller Zellen den CD1b-beschränkten T-Zell-Rezeptor exprimieren; oder
(b) die Transfektion durchgeführt wird, sodass mindestens 30 % aller Zellen den CD1b-beschränkten T-Zell-Rezeptor exprimieren; oder
(c) die Transfektion durchgeführt wird, sodass mindestens 40 % aller Zellen, die den CD1b-beschränkten T-Zell-Rezeptor exprimieren, lebensfähige Zellen sind; oder
(d) die Transfektion durchgeführt wird, sodass mindestens 50 % aller Zellen, die den CD1b-beschränkten T-Zell-Rezeptor exprimieren, lebensfähige Zellen sind; oder
(e) die Transfektion durchgeführt wird, sodass mindestens 60 % aller Zellen, die den CD1b-beschränkten T-Zell-Rezeptor exprimieren, lebensfähige Zellen sind.

10. Zusammensetzung zur Verwendung nach einem der vorhergehenden Ansprüche, wobei mindestens 10⁷ genetisch modifizierte primäre T-Zellen verabreicht werden.

## Revendications

1. Composition à utiliser dans le traitement d'individus infectés par *Mycobacterium tuberculosis,* la composition comprenant une pluralité de lymphocytes T primaires, dans laquelle les lymphocytes T sont stimulés par des anticorps anti-CD3 et anti-CD28 ; dans laquelle
les lymphocytes T stimulés sont transfectés avec un ou plusieurs vecteurs d'acide nucléique recombinants comprenant des acides nucléiques codant pour la chaîne alpha et la chaîne bêta d'un récepteur de lymphocytes T restreint CD1b (TCR) ; et dans laquelle la chaîne alpha comprend SEQ ID NO : 2 et la chaîne bêta comprend SEQ ID NO : 4.

2. Composition à utiliser selon la revendication 1, dans laquelle :
(a) la pluralité de lymphocytes T sont des cellules autologues ; et/ou
(b) la pluralité de lymphocytes T sont stimulés avec des billes modifiées anti CD3-anti CD28.

3. Composition à utiliser selon la revendication 1 ou 2, dans laquelle l'acide nucléique est un ARN recombinant, et dans laquelle la transfection des cellules T stimulées utilise une pluralité de particules polymères qui sont couplées à la molécule d'ARN recombinant.

4. Composition à utiliser selon la revendication 3, dans laquelle la pluralité de particules polymères comprend au moins deux types différents de particules polymères.

5. Composition à utiliser selon la revendication 3 ou la revendication 4, dans laquelle :
(a) la pluralité de particules polymères sont dégradables dans les cellules T stimulées ; ou
(b) la pluralité de particules polymères manquent de fragments de ciblage qui ciblent spécifiquement les particules à un composant du lymphocyte T.

6. Composition à utiliser selon la revendication 3 ou 4, dans laquelle le polymère de la particule polymère est un poly(β-amino ester) ou un chitosane.

7. Composition à utiliser selon la revendication 3 ou 4, dans laquelle la particule polymère est en outre couplée à un deuxième ARNm distinct.

8. Composition à utiliser selon la revendication 7, dans laquelle le deuxième ARNm distinct code pour une enzyme capable de dégrader le polymère de la particule polymère, éventuellement dans laquelle le polymère de la particule polymère est un chitosane et dans laquelle l'enzyme est une chitosanase.

9. Composition à utiliser selon l'une quelconque des revendications 1 à 8, dans laquelle :
(a) la transfection est réalisée de telle sorte qu'au moins 20 % de toutes les cellules expriment le récepteur des lymphocytes T restreints CD1b ; ou
(b) la transfection est réalisée de telle sorte qu'au moins 30 % de toutes les cellules expriment le récepteur des lymphocytes T restreints CD1b ; ou
(c) la transfection est réalisée de telle sorte qu'au moins 40 % de toutes les cellules exprimant le récepteur des lymphocytes T restreints CD1B soient des cellules viables ; ou
(d) la transfection est réalisée de telle sorte qu'au moins 50 % de toutes les cellules exprimant le récepteur des lymphocytes T restreints CD1B soient des cellules viables ; ou
(e) la transfection est réalisée de telle sorte qu'au moins 60 % de toutes les cellules exprimant le récepteur des lymphocytes T restreints CD1B soient des cellules viables.

10. Composition à utiliser selon l'une quelconque des revendications précédentes, dans laquelle au moins 10⁷ lymphocytes T primaires génétiquement modifiés sont administrés.
